# EUROPÄISCHE PATENTSCHRIFT

(11) **EP 0 627 205 B1**
(45) Veröffentlichungstag und Bekanntmachung des Hinweises auf die Patenterteilung: **12.08.1998**
(21) Anmeldenummer: 94107110.2
(22) Anmeldetag: 06.05.1994
(51) Int. Cl.: A61F 5/01

(54) **Sprunggelenkorthese**
Ankle brace
Support pour la cheville

(30) Priorität: 04.06.1993 DE 4318588
(43) Veröffentlichungstag der Anmeldung: 07.12.1994
(73) Patentinhaber: Beiersdorf Aktiengesellschaft, 20245 Hamburg (DE)
(72) Erfinder: Montag, Hans-Jürgen, D-83123 Amerang (DE); Albrod, Andreas, Dr., D-21149 Hamburg (DE); Andrews, Hugh, D-25337 Kölln-Reisiek (DE); Bodenschatz, Stefan, Dr., D-21614 Buxtehude (DE); Rundkowski, Anette, D-22043 Hamburg (DE)
(74) Vertreter: Richter, Werdermann & Gerbaulet

(56) Entgegenhaltungen:
- WO-A-89/10731
- US-A- 2 994 322
- US-A- 4 345 590
- US-A- 4 630 600
- US-A- 5 000 195

## Beschreibung

Die Erfindung betrifft eine Sprunggelenkorthese, insbesondere für die frühfunktionelle Behandlung frischer fibularer Bandrupturen und von schweren Fußwurzeldistorsionen, bestehend aus einer U-förmigen Gelenkmanschette mit einem unterhalb des Fußes verlaufenden Steg aus einem biegsamen Material, wobei die Orthese aus einer Gelenkmanschette mit einer Außenknöchelschiene und einer Innenknöchelschiene besteht, wobei die beiden Knöchelschienen durch einen unter der Ferse des Fußes verlaufenden Steg und auf der Rückseite unter Aussparung der Ferse und des Achillessehnenansatzes durch ein breites Band sowie auf der Vorderseite in ihrem oberen Bereich durch mindestens ein in seiner Länge veränderbares Gurtband miteinander verbunden sind und anatomisch angepaßte und geformte Vertiefungen aufweisen, und aus einem quer unter der Fußsohle proximal der Köpfchen der Mittelfußknochen I-V verlaufenden Mittelfußteil besteht, der die Fußränder lateral und medial umfassen und den Anschluß für zwei Kreuzgurtbänder und ein Quergurtband bilden sowie über das Quergurtband verbunden ist.

Eine solche Sprunggelenkorthese ist aus der im Oberbegriff berücksichtigten WO 89/10731 bekannt. Die bei dieser Sprunggelenkorthese eingesetzten Kreuzgurtbänder sind elastisch ausgebildet und sind lediglich für eine gute Fixierung der Sprunggelenkorthese am Fuß geeignet; sie lassen jedoch Inversions- wie Eversionsbewegungen des Fußes zu.

Die US-A 4,345,590 offenbart eine Stützbandage, bei der mindestens drei längliche Teile mit einem an der Fußsohle angebrachten Teil verbunden sind, wobei von diesen drei länglichen Teilen ein erstes Teil fest an der Innenseite des Fußes, ein zweites Teil fest an der Außenseite des Fußen und ein drittes Teil fest an der Rückseite des Beines angebracht wird, wobei alle drei Teile am Bein anhanftend angebracht sind, um auf diese Weise Verstauchungen des Knöchels zu verhindern und um den Knöchel zu schützen. Diese Bandage besteht aus einem flexiblen Material und soll lediglich ein mediales oder laterales Abwinkeln des Sprunggelenkes verhindern. Um dies zu erreichen besteht die Bandage aus einem Fersenteil und einem Vorderfußteil, die lediglich über ein Steg verbunden sind. Der Vorderfußteil dient nur zum Fixieren der Bandage am Fuß und wirkt nicht einschänkend auf den Bewegungsablauf des Sprunggelenks.

Durch die DE-A-38 40 714 ist eine Sprunggelenkorthese mit einem U-förmigen Stützbügel bekannt, dessen Schenkel unterhalb des Fußes in einem Steg zusammenlaufen, über die Knöchel hinausreichen und in ihrem Endbereich durch ein Befestigungsband zusammen gehalten sind. Dabei ist der äußere Schenkel seitlich vor seinem Knöchel und der innere Schenkel in Gegenüberstellung zum äußeren Schenkel vor der Achillessehne hochgeführt. Beide Schenkel sind in Richtung zu dem Steg bis zu einer Lage vor der Ferse geführt und verlaufen in Richtung zu ihren Enden aufwärts derart, daß sie seitlich neben den Schienbeinkanten etwa parallel zu diesen aufwärts streben, wobei im unteren Bereich der Schenkel ein Halteband angebracht ist, das von dem einen Schenkel über den Rist schräg aufwärts zum anderen Schenkel an diesen festlegbar verläuft, oberhalb der Knöchel um die Achillessehne greift und auf den Rist sich überkreuzend an dem anderen Schenkel in einem Halteteil endet. Mit einer derart ausgebildeten Sprunggelenkorthese soll das Umknicken vor allem in Richtung seitvorn, also in Richtung auf eine Spitzfuß-Stellung, verhindert werden. Da der U-förmige Stützbügel dieser Sprunggelenkorthese mit seinem äußeren Schenkel seitlich vor dem Fußknöchel und mit seinem inneren Schenkel vor der Achillessehne hochgeführt und von einem unterhalb des Fußes verlaufenden Steg zusammengehalten sind, wird der mediale Rand des Mittelfußes nicht erfaßt, so daß der Einsatz dieser Sprunggelenkorthese begrenzt ist.

Eine Fußfixierungsschiene beschreibt die DE-A-39 09 922. Diese Fußfixierungsschiene dient insbesondere zur postoperativen Behandlung eines verletzten Sprunggelenkes mit einem den Fuß umfassenden Fußteil, an den sich nach oben bis in den Wadenbereich reichend ein mit Verschlußzügeln versehener Halteteil anschließt. Der Kalteteil ist dabei in zwei Seitenteile unterteilt, die an den Fußteil anschließen und schalenförmig ausgebildet sind. Jeweils der den Fußknöchel überdeckende Bereich des Seitenteils ist mit einer fensterartigen Ausnehmung versehen. Der Bereich der Achillessehne am Fußteil und am Halteteil ist dabei ausgespart. Die einstell- und feststellbaren bandförmigen Verschlußzügel bestehen aus einem undehnbaren Material, wobei ein Verschlußzügel an dem Fußteil so angeordnet ist, daß er den Fußrücken übergreifend den ersten Strahl des Mittelfußes gegen supinatorisches Aufsteigen fixiert. Mit einer derartigen Fußfixierungsschiene soll zum einen eine einwandfreie Ruhigstellung des zu behandelnden Fußes erreicht werden, und zum anderen sollen die Nachteile eines Gipsverbandes vermieden werden, denn nach Verletzungen und Operationen am äußeren Bandapparat wird oftmals der Fuß zur Ruhig-Stellung eingegipst, wobei eine postoperative Behandlung von Operationswunden wegen zahlreicher gravierender Nachteile nicht möglich ist. Bei dieser Fußfixierungsschiene wird von einer U-förmigen Gelenkmanschette mit vollflächigem Sohlenteil ausgegangen, das den Mittel- und Vorfuß bis zum kleinen Zehenballen erfaßt, wobei jedoch keine ausreichende Gelenkigkeit im Mittelfußbereich gegeben ist.

Nach der US-A- 5 00 195 ist eine Vorrichtung zum Schienen des Knöchels bekannt, die eine feste Winkelausrichtung des Knöchels zum Bein sowie eine Kompression bewirkt, um Schwellungen nach einer Sehnenzerrung oder einem Sehnenriß zu vermindern. Diese Vorrichtung besteht aus einer länglichen, im wesentlichen nicht nachgebenden flachen Körperplatte einschließlich einer ersten und zweiten Endlasche, welche durch eine Mittelzone miteinander verbunden sind, wobei diese Mittelzone so gestaltet ist, daß sie bei angebrachter Schiene unter dem Fußgewölbe liegt, während die Endlaschen so angebracht sind, daß sie an den beiden gegenüberliegenden Seiten des Knöchels aufwärts verlaufen und vom Fußgewölbe aufwärts mindestens über den seitlichen und mittleren Malleolus (Knöchelvorsprung) hinaus den Knöchel bedecken, wobei sich die hinteren Ränder der Endlaschen in einer getrennten, jedoch nebeneinanderliegenden Position entlang der hinteren Seite des Knöchels und die vorderen Ränder der Endlaschen in einer getrennten, jedoch nebeneinanderliegenden Position entlang der vorderen Seite des Knöchels befinden. Die Mittelzone ist von geringerer Breite als die Endlaschen und wird durch eine bogenförmige Vertiefung entlang dem mitteleren Vorderseitenrand der Körperplatte und durch einen Ausschnitt entlang den mittleren Rückseitenrändern der Körperplatte gebildet, wobei sich die bogenförmige Vertiefung am Vorderseitenrand vom oberen Teil der Oberfläche des Fußrückens nach unten und hinten entlang den gegenüberliegenden Seiten des Knöchels erstreckt und so unter dem Fußgewölbe liegt, daß gegen den Knöchel eine Dorsalflexionskraft ausgeübt wird, wenn die Vorderseitenränder der Endlaschen zueinander gezogen werden. Ferner sind elastische Mittel vorgesehen, die die erste und zweite Endlasche entlang ihren jeweiligen Rückseitenrändern von deren obersten Enden bis zu einer Stelle verbinden, die ungefähr neben deren gesteppten Ausschnitten liegt, sowie relativ starre Mittel, die lös- und anpaßbar die erste und zweite Endlasche entlang ihren jeweiligen nebeneinanderliegenden Seitenrändern von deren oberen Enden bis ungefähr zur Mitte des Fußrückens verbinden. Danach umfaßt diese Vorrichtung eine U-förmige Gelenkmanschette mit einem unterhalb des Fußes verlaufenden biegsamen Steg, Außen- und Innenknöchelschienen, die über einen Fersen-Steg miteinander verbunden sind, Vertiefungen zur Knöchelaufnahme, eine rückseitige Fersenaussparung sowie ein breites Verbindungsband zwischen den Schienen und ferner ein längenveränderbares Gurtband auf der Vorderseite im oberen Bereich, jedoch ein Aufbau des Sohlenteils der Vorrichtung aus einem Schienenverbindungssteg und einem weiteren hochflexiblem gelenkig-wirksamen Steg, der in den Mittelfußteil mit seitlichen Laschen zur Gurtbefestigung übergeht, ist bei dieser bekannten Vorrichtung nicht vorgesehen, zumal mit dieser Vorrichtung im wesentlichen nur eine Kompression zum Abklingen von Schwellungen erreicht werden soll.

Der Erfindung liegt die Aufgabe zugrunde, eine Sprunggelenkorthese für eine frühfunktionelle Behandlung frischer fibularer Bandrupturen und von schweren Fußwurzeldistorsionen zu schaffen, mit der eine laterale und mediale Stabilisierung vom oberen und unteren Sprunggelenk erreicht wird, wodurch sowohl Inversionstraumata als auch Eversionstraumata verhindert werden.

Diese Aufgabe wird durch die im Patentanspruch 1 gekennzeichneten Merkmale gelöst.

Nach der erfindungsgemäßen Ausgestaltung besteht die Sprunggelenkorthese aus einer U-förmigen, aus thermoplastischem Material hergestellten Gelenkmanschette, die sich aus einer Außenknöchelschiene und einer Innenknöchelschiene zusammensetzt. Diese Knöchelschienen sind durch einen unter der Ferse verlaufenden Steg, der aus dem gleichen thermoplastischem Material hergestellt ist, verbunden. Die Knöchelschienen weisen ferner anatomisch gerechte Vertiefungen zur Anpassung an die Knöchelkonturen und zum anatomiegerechten Sitz auf. Ein weiteres Element der Orthese ist sein Mittelfußteil, das ebenfalls aus thermoplastischem Material gefertigt ist. Dieser Teil der Orthese läuft quer unter der Fußsohle proximal der Köpfchen der Mittelfußknochen I-V und ist medial und lateral laschenförmig ausgebildet. Die so ausgebildeten Laschen umfassen die Fußränder außen und innen. Sie führen zum einen den Mittelfuß, zum anderen dienen sie zur Befestigung von Kreuz- und Quergurtbändern. Das Mittelfußteil ist dabei fußsohlenseitig durch einen ebenfalls aus thermoplastischem Material hergestellten, jedoch hochflexiblen Steg verbunden. Dieser Steg hat die Funktion eines Gelenkes und arbeitet analog einem Filmscharnier. Die Drehachse dieses in dem hochflexiblen Steg ausgebildeten Gelenkes verläuft von dorso-medial nach antero-lateral und bildet mit der Fußlängsachse einen Winkel von etwa 10°, und zwar entsprechend der Anatomie des unteren Sprunggelenks. Zweckmäßigerweise weist der Gelenksteg auf der medialen Seite eine erhöhte Steifigkeit auf.

Bevorzugterweise sind die aus thermoplastischem Material gefertigten Teile der Sprunggelenktorthese hautseitig derart beschichtet, daß ein inniger Kontakt zur Haut herbeigeführt und während des Tragens der Sprunggelenkorthese erhalten bleibt, und zwar im Sinne der Propriozeption, d.h. in dem Sinne, daß eine ständige Wahrnehmung und Kontrolle der aktuellen Gelenkbelastung und -stellung seitens des Trägers der Orthese gewährleistet ist.

Die Knöchelschienen sind auf der Rückseite unter Aussparung der Ferse und des Achillessehnenansatzes durch ein breites Band miteinander verbunden. Dieses Band zeichnet sich dadurch aus, daß es eine definierte, begrenzte Dehnbarkeit aufweist und somit eine Dislokation nach vorn der Knöchelschienen unter Belastung verhindert.

Auf der Vorderseite sind die Knöchelschienen in ihrem oberen Bereich durch zwei querverlaufende Gurtbänder, die zweckmäßigerweise als Klettbänder ausgebildet sind, miteinander verbunden. Dabei ist jedes Klettband an der lateralen Knöchelschiene angelenkt, wird von dort zur medialen Knöchelschiene geführt, hier durch eine Öse gezogen, lateralwärts gezogen und dann verklettet. Hieraus resultiert eine stufenlose, individuelle Anpassung der Sprunggelenkorthese an die Knöchelgabel und deren Umfang.

Die mediale und laterale Lasche des Mittelfußteils sind durch ein quer über den Fußrücken verlaufendes, ebenfalls als Klettband ausgebildetes Gurtband miteinander verbunden, wodurch eine sichere Fixierung des Mittelfußes in der Sprunggelenkorthese erzielt wird. Dieses Klettband ist an der lateralen Lasche angelenkt, wird an der medialen Lasche durch eine Öse hindurchgeführt und dann verklettet. Dadurch ist eine individuelle Anpassung der Orthese möglich.

An der medialen und lateralen Lasche des Mittelfußteils ist jeweils ein Kreuzgurtband drehbar angelenkt. Auch diese Kreuzgurtbänder sind bevorzugterweise als Klettbänder ausgebildet. Dabei verläuft ein Klettband von der medialen Lasche über den proximalen Fußrücken zu der Außenknöchelschiene, auf deren Außenseite sich eine drehbar gelagerte Öse befindet. Durch diese Öse wird das Klettband gezogen und verklettet. In gleicher Weise wird das andere Klettband von der lateralen Lasche zu der Innenknöchelschiene geführt. Durch diese kreuzzügelförmige Klettbandführung mit stufenloser, individueller Einstellmöglichkeit ergibt sich eine effektive Führung und Zuggurtung des Mittelfußes.

Mit der Sprunggelenkorthese werden folgende Vorteile erreicht:
- Vollständige Umfassung und Führung des gesamten Mittelfußes von medial und lateral.
- Ein anatomiegerechter - die Achsen des oberen und unteren Sprunggelenks berücksichtigender - Gelenksteg zwischen der Gelenkmanschette und dem Mittelfußteil wirkt durch seine mediale Versteifung einem Supinationstrauma zusätzlich entgegen.
- Das die beiden Knöchelschienen auf der Rückseite verbindende elastische Band weist eine definierte, begrenzte Elastizität auf.
- Die Führung der fußrückenseitigen Kreuzgurtbänder bewirkt in Verbindung mit dem Gelenksteg eine sichere Führung und Zuggurtung des Mittelfußes.
- Die hautseitige Beschichtung der Kunststoffteile bedingt einen rutschfesten Sitz der Sprunggelenkorthese am Fuß.

Durch die Konstruktionselemente, wie U-förmige Gelenkmanschette und dem daran angelenkten Mittelfußteil, bewirkt die Sprunggelenkorthese eine laterale und mediale Stabilisierung von oberem und unterem Sprunggelenk, d.h. es werden sowohl Inversionstraumata (Supination, Planatarflexion, Adduktion) als auch Eversionstraumata (Pronation, Dorsalflexion, Abduktion) verhindert. Der Steg am Mittelfußteil mit der medialen Verstärkung verhindert zusätzlich ein Supinationstrauma. Dabei werden die Gelenke in physiologischer Stellung gehalten. Die Sprunggelenkorthese erlaubt einen hohen Grad der Mobilisierung, wie er für die funktionelle Behandlung des Sprunggelenks erforderlich ist; sie garantiert aber auch durch die definierte, begrenzte Elastizität eine sichere Stabilisierung des verletzten Sprunggelenks. Die Sprunggelenkorthese ist deshalb besonders geeignet für frühfunktionelle Behandlung frischer fibularer Bandrupturen sowohl bei primär konservativer Therapie als auch nach operativer Versorgung der Bandverletzung. Des weiteren ist die Sprunggelenkorthese geeignet für die Therapie schwerer Fußwurzeldistorsionen.

Vorteilhafte Ausgestaltungen der Erfindung sind in den Unteransprüchen gekennzeichnet.

Die Erfindung wird anhand schematischer Zeichnungen eines Ausführungsbeispiels näher erläutert. Die Zeichnungen zeigen eine Sprunggelenkorthese in
- Fig. 1: in medialer Ansicht,
- Fig. 2: in frontaler Ansicht,
- Fig. 3: in lateraler Ansicht,
- Fig. 4: in dorsaler Ansicht,
- Fig. 5: in einer Ansicht von oben und
- Fig. 6: in plantarer Ansicht.

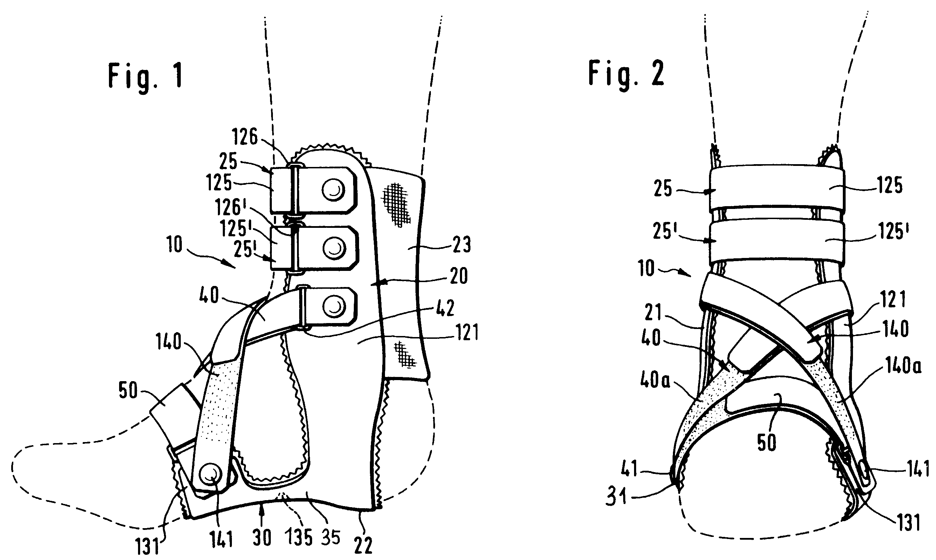

Gemäß Fig. 1 bis 6 besteht die Sprunggelenkorthese 10 aus einer Gelenkmanschette 20 mit einer Außenknöchelschiene 21 und einer Innenknöchelschiene 121. Die beiden Knöchelschienen 21, 121 sind durch einen unter der Ferse des Fußes verlaufenden Steg 22 miteinander verbunden. Rückseitig sind die beiden Knöchelschienen 21, 121 unter Aussparung der Ferse und des Achillessehnenansatzes durch ein elastisches, bevorzugterweise 12 cm breites Band 23 miteinander verbunden, das eine definierte, begrenzte Dehnbarkeit aufweist, damit im angelegten Zustand der Sprunggelenkorthese 10 die beiden Knöchelschienen 21, 121 mit festem Sitz an den beiden Knöchelseiten anliegen. Dieses Band 23 weist in Bezug auf seine Länge eine Querelastizität auf.

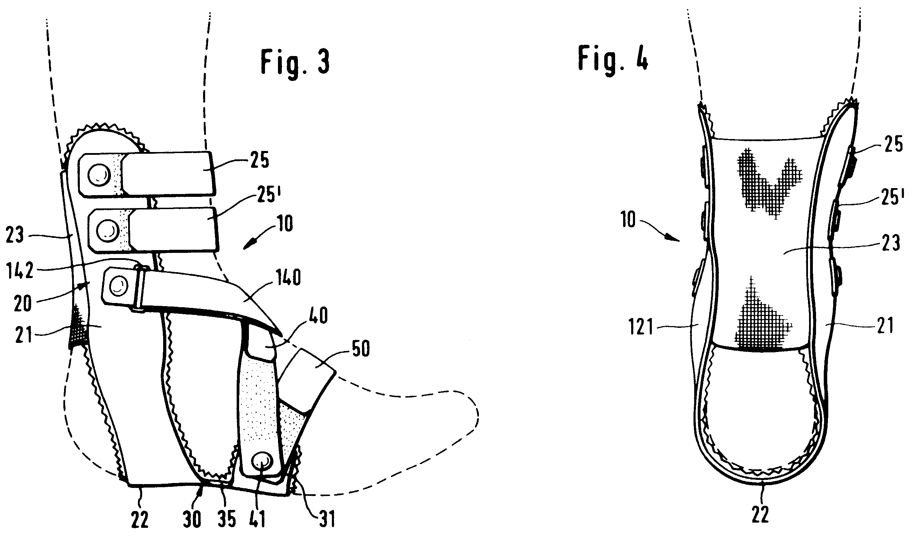

Beide Knöchelschienen 21, 121 weisen anatomisch gerechte Vertiefungen auf, und zwar zur Anpassung an die Knöchelkonturen und zum anatomiegerechten Sitz (Fig. 1 und 3).

Im vorderseitigen Bereich sind die beiden Knöchelschienen 21, 121 der Gelenkmanschette 20 über mindestens ein in seiner Länge veränderbares Gurtband 25 bzw. 25' verbunden, worauf nachstehend noch näher eingegangen wird.

An dem Steg 22 der Gelenkmanschette 20 ist ein Mittelfußteil 30 angeformt, das medial und lateral laschenförmig ausgebildet ist. Dieses Mittelfußteil 30 ist über einen Steg 35, der als Gelenksteg ausgebildet ist, mit dem Steg 22 der Gelenkmanschette 20 verbunden.

Die beiden Laschen 31, 131 des Mittelfußteils 30 umfassen lateral und medial die Fußränder (Fig. 1 und 3). Beide Laschen 31, 131 sind ferner für den Anschluß von zwei Kreuzgurtbändern 40, 140 und für ein Quergurtband 50 ausgebildet. Über das Quergurtband 50 sind die beiden Laschen 31, 131 miteinander verbunden.

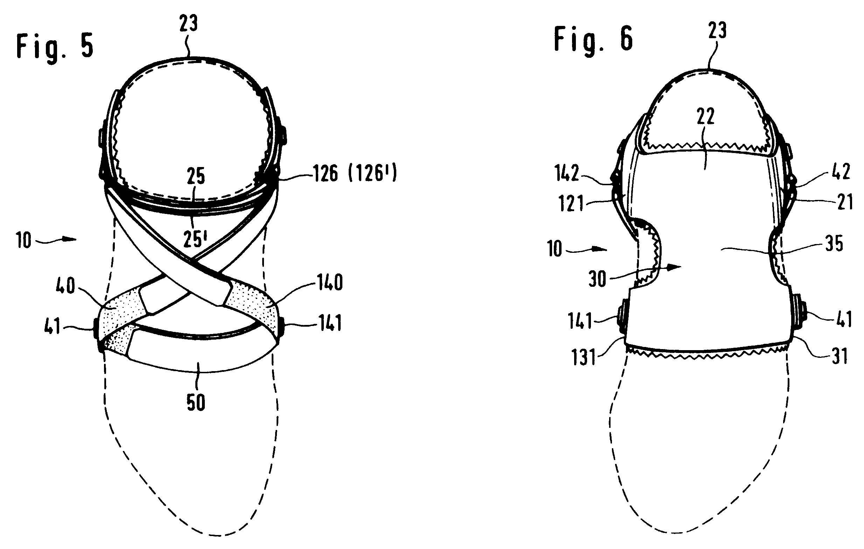

Das Mittelfußteil 30 ist fußsohlenseitig über einen hochflexiblen, als gelenkwirkender Steg 35 mit dem Steg 22 der Gelenkmanschette 20 verbunden. Dieser Gelenksteg 35 weist auf der medialen Seite eine erhöhte Steifigkeit auf (Fig. 6).

An der medialen und lateralen Lasche 31, 131 des Mittelfußteils 30 ist je ein Kreuzgurtband 40, 140 bei 41, 141 angelenkt. Dabei ist das Kreuzgurtband 140 von der medialen Lasche 131 über den proximalen Fußrücken zu der Außenknöchelschiene 21 geführt und an dieser befestigt, während das andere Kreuzgurtband 40 von der lateralen Lasche 31 zu der Innenknöchelschiene 121 geführt und ebenfalls an dieser befestigt ist (Fig. 2). Das Quergurtband 50 ist zweckmäßigerweise mit seinen Enden ebenfalls bei 41, 141 an den Laschen 31, 131 des Mittelfußteiles 30 angelenkt.

Der hochflexible Steg 35 des Mittelfußteils 30 weist bevorzugterweise eine Breite von etwa 5 cm auf. Dabei verläuft die Drehachse dieses vom Steg 35 gebildeten Gelenkes von dorso-medial nach antero-lateral und bildet mit der Fußlängsachse einen Winkel von etwa 10° entsprechend der Anatomie des unteren Sprunggelenks, wobei auch andere Winkelstellungen möglich sind.

Die Wirkung des Steges 35 des Mittelfußteils 30 als Gelenk zu wirken, beruht auf den Umstand, daß der Steg 35 aus einem thermoplastischem Material gefertigt ist, wobei die Gelenkwirkung durch eine quer zur Längsrichtung des Fußes verlaufende Kerbe 135 noch erhöht werden kann, so daß die Funktion eines Filmscharniers erreicht wird. Auf diese Weise wird eine hohe Beweglichkeit des Fußes erreicht (Fig. 1).

Auch die Gelenkmanschette 20 und das Mittelfußteil 30 bestehen aus einem thermoplastischen Material. Die aus dem thermoplastischen Material bestehenden Teile der Sprunggelenkorthese 10 sind hautseitig mit einer Kontakt zur Haut haltenden Beschichtung versehen. Diese Beschichtung weist eine hohe Hautfreundlichkeit auf und kann beispielsweise aus einem Florgewebe oder einem Gewebe mit aufgerauhter Oberfläche bestehen.

Bei dem in Fig. 1 bis 6 gezeigten Ausführungsbeispiel sind die beiden Knöchelschienen 21, 121 der Gelenkmanschette 20 vorderseitig über zwei übereinanderliegend angeordnete Gurtbänder 25, 25' miteinander verbunden. Diese Gurtbänder 25, 25', die gleich ausgebildet sind, bestehen aus querverlaufenden Klettbändern 125, 125'. Diese Klettbänder 125, 125' sind einendseitig an der Außenknöchelschiene 21 angelenkt und von dort zu der Innenknöchelschiene 121 geführt, die Ösen 126, 126' trägt, durch die die Klettbänder hindurchgezogen sind.

Jedes Klettband 125, 125' wird dann mit seinem freien Ende über den Klettverschluß am darunterliegenden Bandende fixiert.

Die an den Laschen 31, 131 des Mittelfußteils 30 befestigten und zu den Knöchelschienen 21, 121 der Gelenkmanschette geführten und auch dort befestigten Kreuzgurtbänder 40, 140 sind in ihren Längen veränderbar und bevorzugterweise als Klettbänder 40a, 140a ausgebildet (Fig. 2). An der lateralen und medialen Lasche 31, 131 des Mittelfußteils 30 ist jeweils ein Klettband 40a bzw. 140a bei 41, 141 drehbar angelenkt. Das Klettband 140a ist von der medialen Lasche 131 zu der Außenknöchelschiene 21 geführt und auf deren Außenseite durch eine drehbar an der Außenknöchelschiene 21 befestigten Öse 42 hindurchgeführt. Das durch die Öse 42 hindurchgeführte Ende des Klettbandes 140a wird dann mit den jeweils darunterliegenden Klettbandabschnitt verklettet.

Das andere Klettband 40a ist von der lateralen Lasche 31 des Mittelfußteils 30 zu der Innenknöchelschiene 121 geführt, auf deren Außenseite eine drehbare Öse 142 befestigt ist, durch die das Klettband 40a hindurchgezogen und entsprechend dem Klettband 140a verklettet ist.

## Patentansprüche

1. Sprunggelenkorthese, bestehend aus einer U-förmigen Gelenkmanschette (20) mit einem unterhalb des Fußes verlaufenden Steg (22) aus einem biegsamen Material, wobei die Orthese (10) aus einer Gelenkmanschette (20) mit einer Außenknöchelschiene (21) und einer Innenknöchelschiene (121) besteht, wobei die beiden Knöchelschienen (21, 121) durch einen unter der Ferse des Fußes verlaufenden Steg (22) und auf der Rückseite unter Aussparung der Ferse und des Achillessehnenansatzes durch ein breites Band (23) sowie auf der Vorderseite in ihrem oberen Bereich durch mindestens ein in seiner Länge veränderbares Gurtband (25; 25') miteinander verbunden sind und anatomisch angepaßte und geformte Vertiefungen aufweisen, und aus einem quer unter der Fußsohle proximal der Köpfchen der Mittelfußknochen I-V verlaufenden Mittelfußteil (30) besteht, der die Fußränder lateral und medial umfassen und den Anschluß für zwei Kreuzgurtbänder (40, 140) und ein Quergurtband (50) bilden sowie über das Quergurtband (50) verbunden ist,
dadurch gekennzeichnet, daß
a. das Mittelfußteil (30) fußsohlenseitig durch einen hochflexiblen, als Gelenk wirkender Steg (35) mit dem Steg (22) der Gelenkmanschette (20) verbunden ist;
b. das Mittelfußteil (30) medial und lateral laschenförmig ausgebildet ist, wobei diese ausgebildeten Laschen (31, 131) die Fußränder lateral und medial umfassen und den Anschluß für die zwei nicht dehnbar ausgebildeten Kreuzgurtbänder (40, 140) und das Quergurtband (50) bilden sowie über das Quergurtband miteinander verbunden sind,
c. wobei eines (140) der beiden Kreuzgurtbänder (40; 140) von der medialen Lasche (131) über den proximalen Fußrücken zu der Außenknöchelschiene (21) geführt und an dieser befestigt ist und das andere Kreuzgurtband (40) von der lateralen Lasche (31) zu der Innenknöchelschiene (121) geführt und an dieser befestigt ist,
d. wobei die Sprunggelenkorthese insbesondere für die frühfunktionelle Behandlung frischer fibularer Bandrupturen und von schweren Fußwurzeldistorsionen geeignet ist.

2. Sprunggelenkorthese nach Anspruch 1, dadurch gekennzeichnet, daß der hochflexible Steg (35) eine Breite von etwa 5 cm aufweist.

3. Sprunggelenkorthese nach einem der Ansprüche 1 und 2, dadurch gekennzeichnet, daß die Drehachse des von dem hochflexiblen Steg (35) gebildeten Gelenkes von dorsomedial nach antero-lateral verläuft und mit der Fußlängsachse einen Winkel von etwa 10° entsprechend der Anatomie des unteren Sprunggelenkes des Fußes bildet.

4. Sprunggelenkorthese nach einem der Ansprüche 1 bis 3, dadurch gekennzeichnet, daß die Gelenkmanschette (20) und das Mittelfußteil (30) aus thermoplastischem Material besteht.

5. Sprunggelenkorthese nach einem der Ansprüche 1 bis 4, dadurch gekennzeichnet, daß die aus thermoplastischem Material bestehenden Teile hautseitig mit einer Kontakt zur Haut haltenden Beschichtung versehen sind.

6. Sprunggelenkorthese nach einem der Ansprüche 1 bis 5, dadurch gekennzeichnet, daß das die beiden Knöchelschienen (21,121) im rückwärtigen Bereich verbindende Band (23) eine Querelastizität aufweist.

7. Sprunggelenkorthese nach einem der Ansprüche 1 bis 6, dadurch gekennzeichnet, daß das Band (23) eine Breite von etwa 12 cm aufweist.

8. Sprunggelenkorthese nach einem der Ansprüche 1 bis 7, dadurch gekennzeichnet, daß die beiden Knöchelschienen (21,121) auf ihrer Vorderseite über zwei Gurtbänder (25,25') miteinander verbunden sind, die als querverlaufende Klettbänder (125,125') ausgebildet sind und die einendseitig an der Außenknöchelschiene (21) angelenkt und von dort zur Innenknöchelschiene (121) geführt und hier durch an der Innenknöchelschiene (121) befestigten Ösen (126,126') hindurchgezogen, lateralwärts gezogen und dann verklettet sind.

9. Sprunggelenkorthese nach einem der Ansprüche 1 bis 8, dadurch gekennzeichnet, daß die an den Laschen (31, 131) des Mittelfußteils (30) befestigten Kreuzgurtbänder (40,140) als Klettbänder (40a,140a) ausgebildet sind, wobei an der medialen und lateralen Lasche (131,31) des Mittelfußteils (30) jeweils ein Klettband (40a;140a) drehbar angelenkt ist, wobei ein Klettband (140a) von der medialen Lasche (131) zu der Außenknöchelschiene (21) verläuft, auf deren Außenseite eine drehbare Öse (42) befestigt ist, durch die das Klettband (140a) gezogen und verklettet ist, wobei das andere Klettband (40a) von der lateralen Lasche (31) zu der Innenknöchelschiene (121) geführt ist, auf deren Außenseite eine drehbare Öse (142) befestigt ist, durch die das Klettband (40a) gezogen und verklettet ist.

10. Sprunggelenkorthese nach eine der Ansprüche 1 bis 9, dadurch gekennzeichnet, daß der das Gelenk bildende Steg (35) des Mittelfußteiles (30) auf der medialen Seite eine erhöhte Steifigkeit aufweist.

## Claims

1. Ankle brace, comprising a U-shaped sleeve joint (20) with a web (22) of a flexible material proceeding underneth the foot, in which the brace (10) is comprised of a joint sleeve (20) with an outer ankle splint (21) and an inner ankle splint (121), in which case two ankle splints (21,121) are interconnected by means of a web (22) proceeding underneath the heel of the foot and, at the rear, while leaving the heel and the Achilles heel tendon attachment uncovered, by means of a wide strap (23) as well as on the front side within its upper area, by means of at least one strap band (25;25') variable in its length, and pos-sess anatomically adapted and configures depressions, and of a metatarsal portion (30) proceeding transversally underneath the sole of the foot proximally to the small condyles of the metatarsal bones I - V, which, laterally and medially, engage around the edges of the foot and form the connection for two cross strap bands (40,140) and a transverse Strap band (50), as well as being connected via the transverse strap band (50),
characterized in that
a. the metatarsal portion (30), on the side of the sole of the foot, is connected by a highly flexible web (35) acting as a joint with the web (22) of the joint sleeve (20);
b. the metatarsal portion (30), medially and laterally, is configured in a tab-like fashion, in which case these shaped tabs (31,131) engage around the foot edges laterally and medially and form the connection for the two non-extensibly constructed cross strap bands (40,140) and the transverse strap band (50) as well as being inter connected via the transverse strap bands;
c. wherein one (140) of the two cross strap bands (40;140) is carried from the medial tab (131) across the proximal back of the foot to the outer ankle splint (21) and is secured to the same and the other cross strap band (40) is carried from the lateral tab (31) to the inner ankle splint (121) and is secured to the same;
d. the ankle brace being particularly suitable for the early functional treatment of fresh fibular ligament ruptures and of severe tarsal distorsions .

2. Ankle brace according to Claim 1,
characterized in that
the highly flexible web (35) possesses a width of approximately 5 cm.

3. Ankle brace according to either Claim 1 or 2,
characterized in that
the axis of rotation of the joint formed by the highly flexible web (35) proceeds from dorsomedial to anterolateral and, with the foot longitudinal axis, forms an angle of approximately 10° according to the anatomy of the lower ankle of the foot.

4. Ankle brace according to any of Claims 1 to 3,
characterized in that
the joint sleeve (20) and the metatarsal portion (30) are comprised of thermoplastic material.

5. Ankle brace according to any of Claims 1 to 4,
charecterized in that,
the parts comprised of thermoplastic material, on the side of the skin, are provided with a coating maintaining contact with the skin.

6. Ankle brace according to any of Claims 1 to 5,
characterized in that
the strap band (23) interconnecting the two ankle splints (21,121) within the rear region possesses a transverse elasticity.

7. Ankle brace according to any of Claims 1 to 6,
characterized in that
the strap band (23) possesses a width of approximately 12 cm.

8. Ankle brace according to any of Claims 1 to 7,
characterized in that
the two ankle splints (21,121), on their front side, are interconnected by means of two strap bands (25, 25'), which are constructed in the form of transversally proceeding Velcro tapes (125,125') and which, at one end, are hinged onto the outer ankle splint (21) and are carried from there to the inner ankle splint (121) and are here drawn through eyelets (126,126') fixed to the inner ankle splint (121), are then drawn sideways and subsequently closed with the aid of Velcro fasteners.

9. Ankle brace according to any of Claims 1 to 8,
characterized in that
the cross strap bands (40,140) fastened to the tabs (31,131) of the metatarsal portion (30) are constructed in the form of Velcro tapes (40a,140a), while to the madial and lateral tab (131,31) of the metatarsal portion (30), one Velcro tape (40a; 140a) each is rotatably hinged, while a Velcro tape (140a) proceeds from the medial tab (131) to the outer ankle splint (21), upon whose outside a rotatable eyelet (42) is attached, through which the Velcro tape (140a) is drawn and Velcro-sealed, whereas the other Velcro tape (40a) is carried from the lateral tab (31) to the inner ankle splint (121), upon whose outside a rotatable eye let (142) is mounted, through which the Velcro tape (40a) is drawn and Velcro-sealed.

10. Ankle brace according to any of Claims 1 to 9,
characterized in that
the web (35) of the metatarsal portion (30) constituting the joint possesses an increased rigidity on the medial side.

## Revendications

1. Support pour la cheville constitué par une manchette d'articulation en forme d'U (20) avec une entretoise (22) en une matière flexible située en dessous du pied, le support (10) étant constitué par une manchette d'articulation (20) avec une attelle de cheville extérieure (21) et une attelle de cheville intérieure (121), les deux attelles de cheville (21, 121) étant reliées l'une a l'autre par une entretoise (22) située sous le talon du pied et sur le côté arrière avec une réservation pour le talon et le début du tendon d'Achille par une large bande (23) ainsi que sur le côté avant dans sa zone supérieure par au moins une sangle (25 ; 25') de longueur réglable et présentant des creux adaptés à l'anatomie et moulés, et par une partie pour le métatarse (30) allant dans le sens transversal sous la plante du pied de manière proximale par rapport aux petites têtes des os du métatarse I-V, partie qui entoure les bords du pied latéralement et médialement et qui forme le raccord pour deux sangles croisées (40, 140) et une sangle transversale (50) et qui est reliée par la sangle transversale (50),
caractérisé en ce que
a. la partie pour le métatarse (30) est reliée du côté de la plante du pied par une entretoise (35) extrêmement flexible, qui agit comme une articulation, à l'entretoise (22) de la manchette d'articulation (20) ;
b. la partie pour le métatarse (30) est configurée médialement et latéralement en forme de collier, ces colliers formés (31, 131) entourant les bords du pied latéralement et médialement et formant le raccord pour les deux sangles croisées (40, 140) configurées non extensibles et la sangle transversale (50) et étant reliées l'une à l'autre par la sangle transversale,
c. l'une des deux sangles croisées (40 ; 140) étant guidée du collier médial (131) par le dos du pied proximal à l'attelle de cheville extérieure (21) et étant fixée à celle-ci et l'autre sangle croisée (40) étant guidée du collier latéral (31) à l'attelle de cheville intérieure (121) et étant fixée à celle-ci,
d. le support de cheville étant approprié en particulier pour le traitement fonctionnel précoce des ruptures de ligaments fibulaires récentes et de graves distorsions du tarse.

2. Support de cheville selon la revendication 1, caractérisé en ce que l'entretoise extrêmement flexible (35) présente une largeur d'environ 5 cm.

3. Support de cheville selon l'une des revendications 1 et 2, caractérisé en ce que l'axe de rotation de l'articulation formée par l'entretoise extrêmement flexible (35) va de dorso-médial à antéro-latéral et forme, avec l'axe longitudinal du pied, un angle d'environ 10° correspondant à l'anatomie de l'articulation tibiotarsienne inférieure du pied.

4. Support de cheville selon l'une des revendications 1 à 3, caractérisé en ce que la manchette d'articulation (20) et la partie pour le métatarse (30) est en matière thermoplastique.

5. Support de cheville selon l'une des revendications 1 à 4, caractérisé en ce que les parties constituées par une matière thermoplastique sont pourvues, côté peau, d'un revêtement qui maintient le contact avec la peau.

6. Support de cheville selon l'une des revendications 1 à 5, caractérisé en ce que la bande (23) qui relie les deux attelles de cheville (21, 121) dans la zone arrière présente une élasticité transversale.

7. Support de cheville selon l'une des revendications 1 à 6, caractérisé en ce que la bande (23) présente une largeur d'environ 12 cm.

8. Support de cheville selon l'une des revendications 1 à 7, caractérisé en ce que les deux attelles de cheville (21, 121) sont reliées l'une à l'autre sur leur côté avant par deux sangles (25, 25') qui sont configurées comme des bandes auto-agrippantes (125, 125') allant dans le sens transversal, qui sont articulées du côté d'une extrémité à l'attelle de cheville extérieure (21) et qui sont guidées en partant de là vers l'attelle de cheville intérieure (121), qui, ici, passent par des oeillets (126, 126') fixés sur l'attelle de cheville intérieure (121), qui sont tendues vers le côté latéral et qui sont ensuite fermées de manière auto-agrippante.

9. Support de cheville selon l'une des revendications 1 à 8, caractérisé en ce que les sangles croisées (40, 140), fixées aux colliers (31, 131) de la partie pour le métatarse (30), sont configurées comme des bandes auto-agrippantes (40a, 140a), une bande auto-agrippante (40a ; 140a) étant respectivement articulée de manière rotative sur le collier médial et sur le collier latéral (131, 31) de la partie pour le métatarse (30), une bande auto-agrippante (140a) allant du collier médial (131) à l'attelle de cheville extérieure (21), sur le côté extérieur de laquelle un oeillet rotatif (42) est fixé, par lequel la bande auto-agrippante (140a) est tendue et fermée de manière auto-agrippante, l'autre bande auto-agrippante (40a) étant guidée du collier latéral (31) à l'attelle de cheville intérieure (121), sur le côté extérieur de laquelle un oeillet rotatif (142) est fixé par lequel la bande auto-agrippante (40a) est tendue et fermée de manière auto-agrippante.

10. Support de cheville selon l'une des revendications 1 à 9, caractérisé en ce que l'entretoise (35) de la partie pour lee métatarse (30) qui forme l'articulation présente, sur le côté médial, une rigidité accrue.
